# EUROPÄISCHE PATENTSCHRIFT

(11) **EP 3 260 154 B1**
(45) Veröffentlichungstag und Bekanntmachung des Hinweises auf die Patenterteilung: **18.12.2019**
(21) Anmeldenummer: 17001045.8
(22) Anmeldetag: 21.06.2017
(51) Int. Cl.: A61M 16/20, A61M 16/00

(54) **BEATMUNGSGERÄT**
VENTILATION DEVICE
RESPIRATEUR

(30) Priorität: 23.06.2016 DE 102016007659
(43) Veröffentlichungstag der Anmeldung: 27.12.2017
(73) Patentinhaber: Löwenstein Medical Technology S.A., 2557 Luxembourg (LU)
(72) Erfinder: Adametz, Benjamin, 22609 Hamburg (DE); Mehnert, Marcel, 25569 Kremperheide (DE)
(74) Vertreter: Marx, Thomas

(56) Entgegenhaltungen:
- EP-A2- 2 153 857
- WO-A1-2010/067236
- DE-A1-102014 012 805
- US-A1- 2012 024 286
- US-A1- 2014 150 793
- US-B1- 6 564 798

## Beschreibung

Die vorliegende Erfindung betrifft eine Beatmungseinrichtung zum Schutz eines Beatmungsgerätes vor rückströmender Ausatemluft, wobei die Beatmungseinrichtung wenigstens eine Gebläseeinrichtung umfasst, die eingerichtet ist, eine Atemgasströmung zu erzeugen und wenigstens eine Strömungsverbindung umfasst, die zu einer Atemschnittstelle geführt ist, wobei die Beatmungseinrichtung eingerichtet ist, die Atemgasströmung anhand wenigstens der Gebläseeinrichtung während einer Einatemphase des Patienten auf wenigstens einen ersten Beatmungsdruck und während einer Ausatemphase auf wenigstens einen zweiten Beatmungsdruck einzustellen.

Bei einer Ventilbeatmung wird in der Regel ein Schlauchsystem mit einem gesteuerten Ausatemventil eingesetzt. Während des Einatmens ist das Ausatemventil geschlossen und während, des Ausatmens ist es üblicherweise offen. Dabei kann die ausgeatmete Luft über das Ventil in die Umgebung strömen.

Allerdings strömt während des Ausatmens die Ausatemluft häufig auch in den Beatmungsschlauch zurück. Dabei kann es zu einer Rückatmung kommen, die über den Beatmungsschlauch hinaus auch bis in das Beatmungsgerät erfolgt. Daran ist besonders problematisch, dass mit der Rückatmung auch Keime in das Gerät gelangen können.

Im Stand der Technik wird ein Rückschlagventil am Geräteeingang angebracht. Dieses Ventil verschließt das Gerät, wenn der Druck der Atemgasströmung soweit absinkt, dass es zu einer Rückatmung in das Gerät kommen könnte.

Allerdings erfordern solche Rückschlagventile oft zeitaufwendige Umbauten am Beatmungsgerät, wenn zwischen einem Schlauchsystem mit Leckagebeatmung und einem Schlauchsystem mit Ventilbeatmung gewechselt werden soll. Das ist bei klinischen Geräten von großem Nachteil, da dort häufig Wechsel vorkommen und die Umbauten somit einen erheblichen Zeitaufwand bedeuten. Auch bei Heimtherapiegeräten bringt der Umbau des Rückschlagventils in der Regel Nachteile mit sich. Beispielsweise erfordert der Austausch einen kostenintensiven Besuch eines Technikers. Der Austauschvorgang kann auch technisch so ausgestaltet werden, dass er von unerfahrenen Benutzern zuverlässig durchgeführt werden kann. Allerdings empfinden viele Benutzer den Umbau oft als lästig.

### Stand der Technik

Aus der DE 10 2014 012 805 A1 sind ein Beatmungsgerät und ein Verfahren zum Betreiben eines Beatmungsgerätes mit einem Gebläse bekannt. Die Beatmungseinrichtung umfasst ein Ausatmenelement, welches ein Ventil umfasst oder als ein solches ausgebildet ist. Das Ausatmenelement ist insbesondere dazu vorgesehen, während der Ausatmung des Benutzers Rückatmung in das Beatmungsgerät zu verhindern.

Aus der US6,564,798 B1 ist ein Verfahren zum Steuern eines Exspirationsventils eines Beatmungsgerätes während einer Exspiration bekannt, wobei das Beatmungsgerät einen inspiratorischen Zweig und einen exspiratorischen Zweig umfasst.

Aus dem Dokument US 2014/0150793 A1 ist ein Verfahren zum Steuern des Betriebs einer CPAP-Vorrichtung bekannt. Die Vorrichtung hat ein Gebläse, eine Patientenschnittstelle, eine Luftzufuhrleitung zum Zuführen von Luft von dem Gebläse zur Patientenschnittstelle, einen Sensor zum Bestimmen des Drucks in der Patientenschnittstelle und einen Steuermechanismus, der bewirkt, dass Luft mit einem gewünschten Druck abgegeben wird.

Aus dem Dokument US 2012/0024286 A1 ist ein Verfahren zum Steuern der Übergänge zwischen zwei unterschiedlichen Drücken, die von einem Atemtherapiegerät geliefert werden, unter Verwendung eines Übergangsdruckprofils, für das die Grundform, die Größe und die Dauer von einem Benutzer des Geräts oder dem Therapeuten des Benutzers gesteuert werden können.

Aus dem Dokument WO2010/067236 A1 ist ein Verfahren zum Bereitstellen einer Druckunterstützung für einen Patienten, dass das Bestimmen einer mit einer Inspirationszeit des Patienten während der Therapie verbundenen Maßnahme umfasst, wobei dem Patienten ein Atemgasstrom mit einem inspiratorischen positiven Atemwegsdruck (IPAP) während mindestens eines Teils davon zugeführt wird eine Einatmungsphase des Patienten zugeführt wird.

Es ist daher die Aufgabe der vorliegenden Erfindung, eine Beatmungseinrichtung und ein Beatmungsgerät zur Verfügung zu stellen, welche einen Schutz vor rückströmender Ausatemluft bieten und dabei besonders bedienerfreundlich sind. Insbesondere" soll ein komfortabler Wechsel zwischen einer Leckagebeatmung und einer Ventilbeatmung möglich sein, ohne dass Umbauten nötig werden.

Diese Aufgabe wird mit der Beatmungseinrichtung des Anspruchs 1 sowie mit dem Beatmungsgerät gemäß Anspruch 17 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Erfindungsgemäß umfasst die Beatmungseinrichtung zumindest eine Überwachungseinrichtung, die eingerichtet ist, zur Überwachung rückströmender Ausatemluft mittels wenigstens einer Sensoreinrichtung wenigstens eine charakteristische Größe für einen Strömungsfluss innerhalb der Strömungsverbindung zu erfassen, wobei die Überwachungseinrichtung eingerichtet ist, wenigsten einen Staudruck gegenüber dem rückströmenden Ausatemfluss durch eine gezielte Erhöhung des Beatmungsdrucks mittels der Gebläseeinrichtung unter Berücksichtigung der erfassten Größe einzustellen, sodass durch den Staudruck einer unerwünschten Rückatmung in die Beatmungseinrichtung entgegengewirkt wird, wobei der Staudruck gleich oder größer als der Druck einer rückströmenden Ausatemluft ist, sodass der Staudruck die Strömung der Ausatemluft in der Strömungsverbindung behindert.

In Ausgestaltung ist die charakteristische Größe für den Strömungsfluss ein Volumendurchfluss.

In einer Weiterbildung ist die Überwachungseinrichtung eingerichtet, unter Berücksichtigung der erfassten Größe die Höhe und/oder die Dauer des Staudrucks und/oder des Zeitpunktes der Erzeugung des Staudrucks einzustellen.

In einer weiteren Weiterbildung ist die Überwachungseinrichtung eingerichtet, den Staudruck zu beaufschlagen, wenn die erfasste Größe einen Strömungsfluss innerhalb der Strömungsverbindung unterhalb eines Schwellenwertes anzeigt und/oder einen negativen Strömungsfluss anzeigt.

In Ausgestaltung weist die Strömungsverbindung eine an die Beatmungseinrichtung koppelbare Schlaucheinrichtung auf und die Überwachungseinrichtung ist eingerichtet, den Staudruck nach einer Latenzzeit zu beaufschlagen, wobei die Latenzzeit die Strömungsdauer des rückströmenden Ausatemflusses durch die Schlaucheinrichtung definiert berücksichtigt.

In weiterer Ausgestaltung ist die Überwachungseinrichtung eingerichtet, die Latenzzeit dynamisch zu errechnen und dabei ein in der Überwachungseinrichtung hinterlegtes Totraumvolumen der Schlaucheinrichtung und/oder wenigstens einen Wert des negativen Strömungsflusses zu berücksichtigen.

In einer Weiterbildung der Erfindung ist die Beatmungseinrichtung in einem Beatmungsgerät angeordnet, wobei das Beatmungsgerät wenigstens ein Ausatemventil aufweist und in einem Betriebsmodus zur Ventilbeatmung betrieben wird und dazu während der Ausatemphase des Patienten das wenigstens eine Ausatemventil zum Abführen von Ausatemluft geöffnet wird.

Voranstehend beschriebene Beatmungseinrichtung, wobei die Überwachungseinrichtung eingerichtet ist, einen Öffnungsgrad des Ausatemventils anzusteuern und wobei der Öffnungsgrad des Ausatemventils (101) und die Einstellung des Staudrucks durch die Überwachungseinrichtung aufeinander abstimmbar sind, sodass eine Spülung der Ausatemluft anpassbar ist.

In einer weiteren Weiterbildung der beiden voranstehend beschriebenen Beatmungseinrichtungen ist die Überwachungseinrichtung eingerichtet, eine Regelung der Einstellung des Staudrucks und/oder eines Öffnungsgrades des einen Ausatemventils in Echtzeit durchzuführen.

In Ausgestaltung ist die Überwachungseinrichtung eingerichtet, bei der Einstellung des Staudrucks auch wenigstens einen Beatmungsparameter und insbesondere der Atemluftbedarf und/oder die Atemfrequenz zu berücksichtigen.

In einer Weiterbildung der Erfindung ist die Überwachungseinrichtung eingerichtet, den zweiten Beatmungsdruck auf wenigstens einen für die Ausatemphase geforderten Solldruck für einen PEEP oder einen EPAP einzustellen.

In einer weiteren Weiterbildung ist die Überwachungseinrichtung eingerichtet, den zweiten Beatmungsdruck auf den Solldruck zu einem anderen Zeitpunkt als die Anhebung des Beatmungsdrucks auf den Staudruck einzustellen.

In einer weiteren Weiterbildung ist die Überwachungseinrichtung eingerichtet, den Solldruck gegenüber dem Staudruck priorisiert einzustellen.

In Ausgestaltung ist die Überwachungseinrichtung eingerichtet, die Höhe und/oder Dauer und/oder den Zeitpunkt des Staudrucks unter Berücksichtigung des Solldrucks einzustellen.

In weiterer Ausgestaltung ist die Beatmungseinrichtung in einem Beatmungsgerät angeordnet, wobei das Beatmungsgerät in einem Betriebsmodus zur Ventilbeatmung ohne ein Rückschlagventil zur Verhinderung von rückströmender Ausatemluft betrieben wird.

In einer Weiterbildung der Erfindung wird die Strömungsverbindung durch wenigstens eine an die Beatmungseinrichtung koppelbare Schlaucheinrichtung und durch wenigstens einen in der Beatmungseinrichtung aufgenommen Geräteabschnitt bereitgestellt und wobei die Überwachungseinrichtung eingerichtet ist, die charakteristische Größe zu erfassen und/oder den Staudruck im Geräteabschnitt zu beaufschlagen.

In einem Beispiel, der vorliegenden Erfindung ist das Beatmungsgerät eingerichtet, ein Verfahren auszuführen, das zum Schutz einer Beatmungseinrichtung eines Beatmungsgerätes vor rückströmender Ausatemluft dient.

Dabei wird bei dem beispielhaften Verfahren mit wenigstens einer Gebläseeinrichtung wenigstens eine Atemgasströmung erzeugt und über wenigstens eine Strömungsverbindung zu einer Atemschnittstelle geführt. Die Atemgasströmung wird anhand wenigstens einer Steuereinrichtung während einer Einatemphase des Patienten auf wenigstens einen ersten Beatmungsdruck eingestellt. Während einer Ausatemphase wird die Atemgasströmung auf wenigstens einen zweiten Beatmungsdruck eingestellt. Dabei wird zur Überwachung rückströmender Ausatemluft wenigstens eine charakteristische Größe für einen Strömungsfluss innerhalb der Strömungsverbindung erfasst. Die Überwachung erfolgt mittels wenigstens einer Überwachungseinrichtung. Es wird wenigstens ein Staudruck gegenüber dem rückströmenden Ausatemfluss durch eine gezielte Erhöhung des Beatmungsdrucks unter Berücksichtigung der erfassten Größe eingestellt. Durch den Staudruck wird einer unerwünschten Rückatmung in die Beatmungseinrichtung entgegengewirkt.

Das beispielhafte Verfahren hat viele Vorteile. Ein erheblicher Vorteil ist, dass der Schutz vor rückströmender Ausatemluft mittels eines Staudrucks erfolgt, welcher durch die Gebläseeinrichtung aufgebaut wird. Dadurch kann auf ein Rückschlagventil zur Verhinderung einer Rückatmung in die Beatmungseinrichtung verzichtet werden. Somit ermöglicht das erfindungsgemäße Verfahren einen besonders bedienerfreundlichen und komfortablen Wechsel zwischen einer Leckagebeatmung und einer Ventilbeatmung, da keine Umbauten bezüglich eines Rückschlagventils vorgenommen werden müssen.

Im Rahmen der vorliegenden Erfindung wird unter einem Staudruck insbesondere ein Druck verstanden, welcher gleich oder größer als der Druck einer rückströmenden Ausatemluft ist, sodass der Staudruck die Strömung der Ausatemluft in der Schlaucheinrichtung behindert.

Es ist möglich, dass der zweite Beatmungsdruck geringer als der erste Beatmungsdruck ist. Möglich ist auch, dass der zweite Beatmungsdruck höher als der erste Beatmungsdruck ist. Insbesondere wird der zweite Beatmungsdruck nur zeitweise gezielt auf den Staudruck erhöht. Möglich ist auch, dass der zweite Beatmungsdruck während seiner gesamten Dauer auf den Staudruck erhöht wird.

Es ist möglich, dass der Staudruck geringer als der erste Beatmungsdruck ist. Möglich ist auch, dass der Staudruck höher als der erste Beatmungsdruck ist. Insbesondere wird der Staudruck nur zweitweise erhöht. Möglich ist auch, dass der Staudruck dem zweiten oder ersten Beatmungsdruck zumindest zeitweise entspricht.

In dem beispielhaften Verfahren wird besonders bevorzugt als charakteristische Größe für den Strömungsfluss ein Volumendurchfluss erfasst. Ein Volumendurchfluss eignet sich zur Überwachung eines zurückströmenden Ausatemflusses besonders gut. Möglich ist auch, dass als charakteristische Größe für den Strömungsfluss eine Strömungsgeschwindigkeit und/oder ein Massendurchfluss und/oder eine Strömungsrichtung erfasst wird. Es kann auch ein Druck erfasst werden. Insbesondere wird ein in die Strömungsverbindung zurückströmender exspiratorischer Fluss des Patienten überwacht.

Vorzugsweise werden unter Berücksichtigung der erfassten Größe die Höhe und/oder die Dauer des Staudrucks und/oder der Zeitpunkt der Zeugung des Staudrucks eingestellt. Eine solche Einstellung in Abhängigkeit der erfassten Größe ermöglicht eine besonders gezielte Anpassung des Staudrucks. Beispielsweise wird die Höhe des Staudrucks so eingestellt, dass dessen Druck höher oder gleich dem Druck der rückströmenden Ausatemluft ist. Die Einstellung des Staudrucks erfolgt insbesondere anhand einer Einstellung der Gebläsedrehzahl der Gebläseeinrichtung.

In dem beispielhaften Verfahren ist es auch möglich, dass der Staudruck auf einen Druck eingestellt wird, welcher um einen bestimmten Faktor größer ist als der Druck der rückströmenden Ausatemluft. Der Faktor wird vorzugsweise so gewählt, dass eine Rückströmung in eine Schlaucheinrichtung möglich ist, die Ausatemluft aber nicht in die Beatmungseinrichtung eintritt. Die Dauer des Staudrucks wird vorzugsweise so eingestellt, dass einer Rückströmung in die Beatmungseinrichtung bis zum Ende der jeweiligen Ausatemphase entgegengewirkt wird. Der Zeitpunkt der Erzeugung des Staudrucks wird insbesondere so eingestellt, dass die rückströmende Ausatemluft spätestens dann durch den Staudruck gehindert wird, wenn diese in die Beatmungseinrichtung eintreten würde.

In dem beispielhaften Verfahren wird der Staudruck dann beaufschlagt, wenn die erfasste Größe einen Strömungsfluss innerhalb der Strömungsverbindung unterhalb wenigstens eines Schwellenwertes anzeigt. Vorzugsweise definiert der Schwellenwert eine Verlangsamung der von der Gebläseeinrichtung erzeugten Atemgasströmung, die zu einer Rückatmung in das in die Beatmungseinrichtung führt. Der Staudruck kann auch dann beaufschlagt werden, wenn die erfasste Größe einen negativen Strömungsfluss anzeigt. Möglich ist auch, dass der Staudruck dann beaufschlagt wird, wenn die erfasste Größe einen Strömungsstillstand der von der Gebläseeinrichtung erzeugten Atemgasströmung innerhalb der Strömungsverbindung anzeigt. Ein negativer Strömungsfluss ist insbesondere dadurch gekennzeichnet, dass die Strömungsrichtung von der Atemschnittstelle in Richtung Gebläseeinrichtung verläuft.

In dem beispielhaften Verfahren ist es möglich, dass der Staudruck nach wenigstens einer Latenzzeit beaufschlagt wird. Die Latenzzeit definiert vorzugsweise die Strömungsdauer des rückströmenden Ausatemflusses durch eine an die Beatmungseinrichtung gekoppelte Schlaucheinrichtung. Insbesondere ist die Strömungsdauer des rückströmenden Ausatemflusses neben dem Totraumvolumen auch von der Strömungsgeschwindigkeit bzw. dem Strömungsdruck abhängig. Die Strömungsgeschwindigkeit bzw. der Strömungsdruck wird vorzugsweise durch die Überwachungseinrichtung und besonders bevorzugt anhand der charakteristischen Größe erfasst.
Eine solche Latenzzeit hat den Vorteil, dass der Staudruck beispielsweise nicht während der gesamten Ausatemphase aufrechterhalten werden muss. Dadurch wird der Einfluss des Staudrucks auf die Beatmung besonders geringgehalten und zugleich ein wirkungsvoller Schutz der Beatmungseinrichtung vor Kontamination gewährleistet. Ein Eintreten des rückströmenden Ausatemflusses in die Schlaucheinrichtung ist in der Regel hinsichtlich einer Kontaminierung unproblematisch, da die Schlaucheinrichtungen entweder Einwegsysteme sind oder unaufwendig gereinigt werden können.

In dem beispielhaften Verfahren ist es auch möglich, dass die Latenzzeit einen Zeitraum bis zum Eintreffen des rückströmenden Ausatemflusses an einer zu schützenden Position der Strömungsverbindung definiert. Die zu schützende Position der Strömungsverbindung liegt insbesondere an einem fest installierten Geräteabschnitt im Inneren der Beatmungseinrichtung.
Die Latenzzeit beginnt vorzugsweise zu einem Zeitpunkt, wenn die erfasste Größe einen negativen Strömungsfluss anzeigt und/oder ein Schwellenwert für den Strömungsfluss unterschritten wird. Ein negativer Strömungsfluss umfasst beispielsweise einen Atemgasanteil und einem Ausatemluftanteil. Die Erkennung eines negativen Strömungsflusses anhand der charakteristischen Größe bedeutet in der Regel zunächst eine Rückströmung des Atemgasanteils. Zu diesem Zeitpunkt beginnt vorzugsweise die Latenzzeit. Erreicht nun der Ausatemluftanteil die kritischen Bereiche der Strömungsverbindung und beispielsweise den Geräteabschnitt, ist die Latenzzeit verstrichen und der Staudruck wird beaufschlagt. So ist eine wirkungsvolle Verhinderung von kontaminierter Ausatemluft in die Beatmungseinrichtung gewährleistet.

In dem beispielhaften Verfahren wird die Latenzzeitvorzugsweise durch die Überwachungseinrichtung dynamisch errechnet. Dabei wird insbesondere ein in der Überwachungseinrichtung hinterlegtes Totraumvolumen einer angekoppelten Schlaucheinrichtung berücksichtigt. Vorzugsweise wird auch wenigstens ein Wert des negativen Strömungsflusses berücksichtigt. Der Wert des negativen Strömungsflusses ist vorzugsweise die Strömungsgeschwindigkeit und/oder der Strömungsdruck und/oder besonders bevorzugt der Volumendurchfluss. Durch eine solche Berechnung der Latenzzeit kann die Beaufschlagung der Staudrucks gezielt an die eingesetzte Schlaucheinrichtung und an die Rückströmung angepasst werden. Die Rückströmung hängt beispielsweise von der Ausatemtätigkeit des Patienten sowie auch von dem zweiten Beatmungsdruck während der Ausatemphase ab. Der zweite Beatmungsdruck außerhalb der Erhöhung auf den Staudruck wird beispielsweise unter Berücksichtigung eines PEEP (Positive End-Expiratory Pressure) oder eines EPAP (Expiratory Positive Airway Pressure) eingestellt.

In allen Ausgestaltungen des beispielhaften Verfahrens ist es besonders bevorzugt, dass das Beatmungsgerät in einem Betriebsmodus zur Ventilbeatmung betrieben wird. Dazu wird insbesondere während der Ausatemphase des Patienten wenigstens ein Ausatemventil zum Abführen von Ausatemluft geöffnet. Der zweite Beatmungsdruck dient vorzugsweise auch zum Ausspülen ausgeatmeter Luft aus einer Atemschnittstelle. Während der Ausatemphase und/oder während des zweiten Beatmungsdruckes ist das Ausatemventil vorzugsweise wenigstens teilweise geöffnet. Insbesondere umfasst das Beatmungsgerät wenigstens eine Schlaucheinrichtung mit einem steuerbaren Ausatemventil. Die Beatmungseinrichtung ist insbesondere dazu geeignet und ausgebildet, das Ausatemventil zu steuern und insbesondere über einen PEEP-Steuerdruck und/oder einen EPAP-Steuerdruck zu steuern.

In einer solchen Ausgestaltung ist das beispielhafte Verfahren besonders vorteilhaft, da bei einer Ventilbeatmung besonders häufig eine Rückatmung in die Schlaucheinrichtung auftritt und es somit auch zu einer Rückatmung in die Beatmungseinrichtung kommen kann. Daher ist im Stand der Technik bei einer Ventilbeatmung in der Regel ein Rückschlagventil zwischen der Schlaucheinrichtung und der Beatmungseinrichtung vorgesehen. Durch das Beaufschlagen mit dem Staudruck kann bei einer Ventilbeatmung daher auf das Rückschlagventil verzichtet werden. Möglich ist auch, dass das Beatmungsgerät in einem Betriebsmodus zur Leckagebeatmung betrieben wird. In einem solchen Modus wird vorzugsweise kein Staudruck erzeugt. Es kann aber auch vorgesehen sein, dass ein kontinuierlicher Beatmungsdruck zeitweise auf den Staudruck erhöht wird. Möglich ist aber auch, dass auch in einem Modus zur Leckagebeatmung ein erster und ein zweiter Beatmungsdruck vorgesehen sind und der zweite Beatmungsdruck auf einen Staudruck erhöht wird oder ergänzend ein Staudruck bereitgestellt wird.

Insbesondere ist ein Öffnungsgrad des Ausatemventils steuerbar. Vorzugsweise wird der Öffnungsgrad des Ausatemventils durch die Überwachungseinrichtung gesteuert. Insbesondere umfasst das Steuern auch ein Regeln. Insbesondere ist der Öffnungsgrad des Ausatemventils über einen PEEP-Steuerdruck und/oder einen EPAP-Steuerdruck steuerbar.

Bevorzugt werden der Öffnungsgrad des Ausatemventils und die Einstellung des Staudrucks durch die Überwachungseinrichtung aufeinander abgestimmt. Insbesondere wird der Staudruck in Abhängigkeit des Öffnungsgrades angepasst. Der Öffnungsgrad kann auch in Abhängigkeit des Staudrucks eingestellt werden. Insbesondere ist die Abstimmung so ausgelegt, dass eine Spülung der Ausatemluft aus der Atemschnittstelle angepasst wird. Besonders bevorzugt wird durch die Abstimmung eine möglichst vollständige Spülung der Ausatemluft erreicht. Dabei ist möglich, dass der Staudruck auch als ein Spüldruck zur Spülung der Ausatemluft eingesetzt wird.

In allen Ausgestaltungen des beispielhaften Verfahrens ist es besonders bevorzugt, dass eine Regelung der Einstellung des Staudrucks und/oder eines Öffnungsgrades des Ausatemventils in Echtzeit durchgeführt werden. Insbesondere umfasst die Echtzeitregelung auch die Erfassung und/oder Auswertung der charakteristischen Größe für den Strömungsfluss innerhalb der Strömungsverbindung. Eine Echtzeitregelung ermöglicht eine besonders zügige Einstellung des Staudrucks. Dadurch kann zum einen wirkungsvoll vor Kontamination geschützt werden und zum anderen wird durch die schnelle Reaktionsfähigkeit des Beatmungssystems ein hohes Maß an Beatmungsqualität erreicht.

Besonders bevorzugt ist eine Gebläseeinrichtung mit einem hochdynamischen Gebläse vorgesehen.

Bei der Einstellung des Staudrucks wird vorzugsweise auch wenigstens ein Beatmungsparameter berücksichtigt. Besonders bevorzugt wird der Atemluftbedarf und/oder die Atemfrequenz berücksichtigt. Es können auch andere Beatmungsparameter berücksichtigt werden, wie zum Beispiel ein notwendiger minimaler bzw. maximaler Beatmungsdruck. Besonders bevorzugt werden auch der erste Beatmungsdruck und/oder der zweite Beatmungsdruck in Abhängigkeit wenigstens eines für die Beatmung geforderten Beatmungsparameter eingestellt bzw. geregelt. So kann eine optimale Beatmungsqualität gewährleistet werden kann. Insbesondere wird der zweite Beatmungsdruck auf wenigstens einen für die Ausatemphase geforderten Solldruck eingestellt wird. Der geforderte Solldruck entspricht vorzugsweise einem PEEP oder einem CPAP. Ein solcher Solldruck wird insbesondere bei einer Ventilbeatmung eingestellt.

Dabei erfolgt die Einstellung des zweiten Beatmungsdrucks auf den Solldruck insbesondere zu einem anderen Zeitpunkt als die Anhebung auf den Staudruck. Insbesondere erfolgt die Anhebung des zweiten Beatmungsdrucks auf den Staudruck zeitlich vor oder nach der Einstellung des zweiten Beatmungsdrucks auf den Solldruck. Möglich ist auch, dass eine Einstellung des zweiten Beatmungsdrucks auf den Sölldruck durch die Einstellung auf den Staudruck zeitweise unterbrochen wird.
Es kann auch vorgesehen sein, dass die Einstellung des zweiten Beatmungsdrucks auf den Solldruck gleichzeitig mit der Anhebung des zweiten Beatmungsdrucks auf den Solldruck erfolgt, wenn die notwendigen Druckniveaus gleich oder hinreichend ähnlich sind. Es ist möglich, dass der Solldruck gegenüber dem Staudruck priorisiert eingestellt wird. Beispielsweise kann der Staudruck geringer eingestellt werden, wenn der Solldruck sonst ungünstig abgesenkt bzw. angehoben werden würde. Es kann auch vorgesehen sein, dass der Staudruck zugunsten eines geforderten Solldrucks vorübergehend auf einen Wert eingestellt wird, welcher eine Rückströmung des Ausatemflusses in die Beatmungseinrichtung zulässt. Durch eine solche Priorisierung kann stets eine sichere und zuverlässige Beatmung gewährleistet werden. Es kann auch vorgesehen sein, dass der Staudruck priorisiert gegenüber dem Solldruck eingestellt wird.

Vorzugsweise werden die Höhe und/oder die Dauer und/oder der Zeitpunkt des Staudrucks unter Berücksichtigung des Solldrucks für den zweiten Beatmungsdruck eingestellt. So können Höhe bzw. Dauer oder Zeitpunkt des Staudrucks so gewählt werden, dass der notwendige Solldruck des zweiten Beatmungsdrucks eingehalten werden kann. Dadurch ist die Bereitstellung des Solldrucks möglich, ohne dass die Einstellung auf den geforderten Solldruck ungünstig beeinflusst würde.

In allen Ausgestaltungen des beispielhaften Verfahrens ist es besonders bevorzugt, dass das Beatmungsgerät in einem Betriebsmodus zur Ventilbeatmung ohne ein Rückschlagventil zur Verhinderung von rückströmender Ausatemluft betrieben wird. Dadurch kann besonders komfortabel und unaufwendig zwischen einer Ventilbeatmung und einer Leckagebeatmung gewechselt werden, da kein Rückschlagventil ausgetauscht werden muss.

Vorzugsweise wird die Strömungsverbindung durch wenigstens eine Schlaucheinrichtung sowie durch wenigstens einen Geräteabschnitt bereitgestellt. Die Schlaucheinrichtung ist dabei als eine an die Beatmungseinrichtung koppelbare Schlaucheinrichtung ausgebildet. Der Geräteabschnitt ist insbesondere in der Beatmungseinrichtung aufgenommen und vorzugsweise fest installiert. Insbesondere umfasst die Beatmungseinrichtung wenigstens eine Kupplungseinrichtung zur Anbindung der Schlaucheinrichtung. Der Geräteabschnitt liegt dann insbesondere zwischen Gebläseeinrichtung und Kupplungseinrichtung. Der Geräteabschnitt kann die Kupplungseinrichtung umfassen. An der Schlaucheinrichtung ist insbesondere das Ausatemventil angeordnet.

Die charakteristische Größe für den Strömungsfluss wird insbesondere im Geräteabschnitt erfasst. Der Geräteabschnitt kann dazu wenigstens einen Messkanal mit wenigstens einem Sensor umfassen. Vorzugsweise wird auch der Staudruck im Geräteabschnitt beaufschlagt. Insbesondere ist der Staudruck so eingestellt, dass ein Eintreten des rückströmenden Ausatemflusses in den Geräteabschnitt entgegengewirkt wird. Ein rückströmender Ausatemfluss in der Beatmungseinrichtung ist besonders ungünstig, da diese in der Regel nur mit sehr hohem Aufwand gereinigt werden kann. Hingegen ist eine Rückströmung in die Schlaucheinrichtung eher unproblematisch.

Des Weiteren betrifft die vorliegende Erfindung ein Beatmungsgerät umfassend wenigstens eine voranstehend beschriebene Beatmungseinrichtung.

Das erfindungsgemäße Beatmungsgerät umfasst wenigstens eine Beatmungseinrichtung. Die Beatmungseinrichtung umfasst wenigstens eine Gebläseeinrichtung zur Erzeugung einer Atemgasströmung. Die Beatmungseinrichtung umfasst wenigstens eine Strömungsverbindung, über welche die Atemgasströmung von der Gebläseeinrichtung zu einer Atemschnittstelle abführbar ist. Die Atemschnittstelle dient insbesondere zur Ankopplung des Beatmungsgerätes an eine Atemöffnung des Patienten. Die Beatmungseinrichtung ist dazu geeignet und ausgebildet, die Atemgasströmung anhand der Gebläseeinrichtung während einer Einatemphase eines Patienten auf wenigstens einen ersten Beatmungsdruck einzustellen. Die Beatmungseinrichtung ist dazu geeignet und ausgebildet, die Atemgasströmung während einer Ausatemphase auf wenigstens einen zweiten Beatmungsdruck einzustellen. Dabei umfasst die Beatmungseinrichtung wenigstens eine Überwachungseinrichtung zur Überwachung rückströmender Ausatemluft. Die Überwachungseinrichtung ist dazu geeignet und ausgebildet, mittels wenigstens einer Sensoreinrichtung wenigstens eine charakteristische Größe für einen Strömungsfluss innerhalb der Strömungsverbindung zu erfassen. Die Überwachungseinrichtung ist dazu geeignet und ausgebildet, unter Berücksichtigung der erfassten Größe wenigstens einen Staudruck gegenüber dem rückströmenden Ausatemfluss durch eine gezielte Erhöhung des zweiten Beatmungsdrucks mittels der Gebläseeinrichtung bereitzustellen.

Auch das erfindungsgemäße Beatmungsgerät bietet viele Vorteile. Besonders vorteilhaft ist, dass auf ein Rückschlagventil zur - Verhinderung von rückströmender Ausatemluft verzichtet werden kann. Dadurch kann zwischen einer Leckagebeatmung und einer Ventilbeatmung gewechselt werden, ohne dass ein Rückschlagventil eingebaut bzw. ausgebaut werden muss. Der Benutzer bzw. Betreuer muss lediglich das entsprechende Schlauchsystem mit dem gewünschten Ausatemsystem an das Beatmungsgerät anschließen.

Das Beatmungsgerät umfasst insbesondere wenigstens ein steuerbares Ausatemventil zum Abführen von Ausatemluft. Insbesondere ist der Öffnungsgrad des Ausatemventils durch die Überwachungseinrichtung steuerbar. Die Überwachungseinrichtung ist insbesondere dazu geeignet und ausgebildet, den Öffnungsgrad des Ausatemventils und/oder die Einstellung des Staudrucks in Echtzeit zu regeln. Insbesondere umfasst das Beatmungsgerät kein Rückschlagventil zur Verhinderung einer Rückatmung in die Beatmungseinrichtung.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
Fig. 1 eine schematische Darstellung eines erfindungsgemäßen Beatmungsgerätes mit verschiedenen Schlaucheinrichtungen in einer perspektivischen Ansicht;
Fig. 2 eine stark schematische Darstellung eines erfindungsgemäßen Beatmungsgerätes;
Fig. 3 ein stark skizzierter Druckverlauf ohne Staudruck; und
Fig. 4 ein stark skizzierter Druckverlauf mit Staudruck.

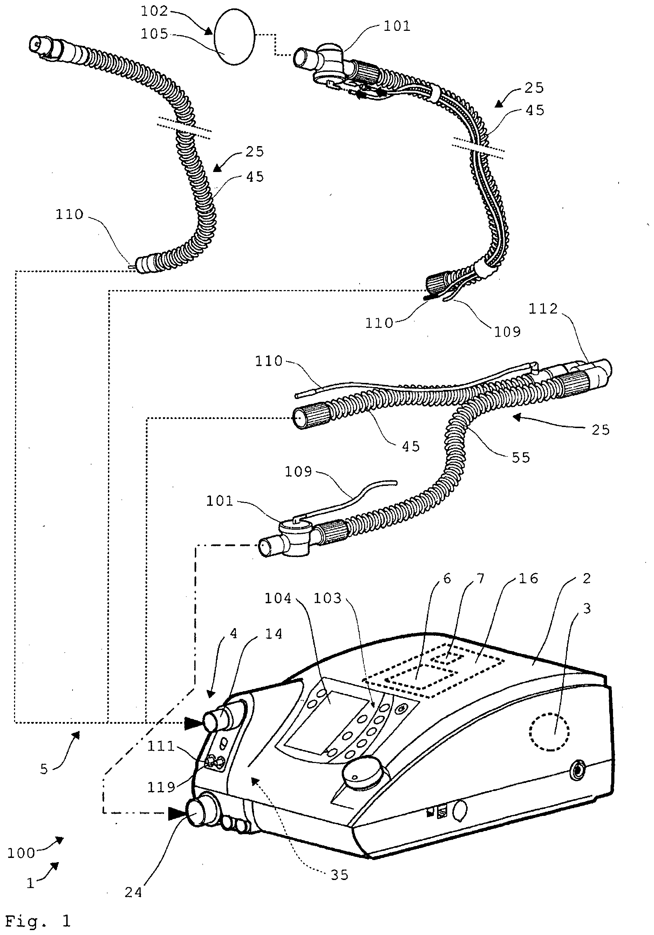

Die Figur 1 zeigt ein erfindungsgemäßes Beatmungsgerät 1, welches als ein Heimbeatmungsgerät 100 bzw. Schlaftherapiegerät ausgebildet ist. Das Beatmungsgerät 1 kann aber auch als klinisches Beatmungsgerät 1 eingesetzt werden. Das Beatmungsgerät 1 wir hier nach dem beispielhaften Verfahren betrieben.

Das Beatmungsgerät 1 umfasst eine Beatmungseinrichtung 2 mit einer Gebläseeinrichtung 3 zur Erzeugung einer Atemgasströmung für die Beatmung. Zudem sind beispielhaft drei verschiedene Typen von Schlaucheinrichtungen 25 gezeigt, welche an die Beatmungseinrichtung 2 anschließbar sind. Die angeschlossene Schlaucheinrichtung 25 bildet zusammen mit einem hier nicht sichtbaren Geräteabschnitt 35 im Inneren der Beatmungseinrichtung 2 eine Strömungsverbindung 5 zwischen Gebläseeinrichtung 3 und einer Atemschnittstelle 102.
Zur Steuerung bzw. Regelung der Beatmungseinrichtung 2 und insbesondere der Gebläseeinrichtung 3 ist hier eine Steuereinrichtung 16 vorgesehen. Die Bedienung und Einstellung des Beatmungsgerätes 1 erfolgt über eine Bedieneinrichtung 103 mit einer Anzeigeeinrichtung 104.

Die Steuereinrichtung 16 ist zudem mit einer hier nicht näher dargestellten Sensoreinrichtung 7 wirkverbunden. Die Sensoreinrichtung 7 umfasst einen oder mehrere Sensoren 17, 27 zur Erfassung von Beatmungsparametern und vorzugsweise von charakteristischen Größe (6')n der Atemgasströmung.

Die Steuereinrichtung 16 stellt z. B. einen notwendigen Minimaldruck bereit und kompensiert Druckschwankungen, die durch die Atemtätigkeit des Benutzers bedingt sind. Beispielsweise erfasst die Steuereinrichtung 16 anhand der Sensoreinrichtung 7 auch den gegenwärtigen Druck und Flow der Beatmung und regelt die Leistung der Gebläseeinrichtung 3 entsprechend nach, bis ein gewünschter Beatmungsdruck bzw. Flow anliegt.

Die zur Einstellung der Beatmungseinrichtung 2 bzw. der Gebläseeinrichtung 3 benötigten Beatmungsparameter sowie die Gerätekonfiguration und/oder Gerätesoftware sind in einer Speichereinrichtung der Steuereinrichtung 16 hinterlegt.
Das hier gezeigte Beatmungsgerät 1 kann als ein Fix-Level-Gerät oder auch als ein Automatic-Level-Gerät ausgebildet sein. Insbesondere erfolgt dabei durch die Steuereinrichtung 16 eine Regelung auf Soll-Beatmungsparameter, welche zuvor anhand der charakteristischen Atmung eines Benutzers individuell berechnet und festgelegt worden sind.

Die Beatmungseinrichtung 2 wird hier dynamisch und insbesondere je nach Atemphase des Benutzers angepasst. Beispielsweise kann anhand der Steuereinrichtung 16 bzw. Sensoreinrichtung 7 ein Atemphasenwechsel erkannt werden, sodass je nach Atemphase ein höherer bzw. niedrigerer Druck bereitgestellt werden kann. So wird während einer Einatemphase des Patienten die Atemgasströmung auf einen ersten Beatmungsdruck und während einer Ausatemphase auf einen zweiten Beatmungsdruck eingestellt wird. Insbesondere reagiert das Beatmungsgerät 1 auf bestimmte Atemereignisse, wie z. B. Schnarchen, Atemabflachungen und/oder obstruktive Druckspitzen mit entsprechenden Einstellungen der Beatmungsparameter.

Um bei der Beatmung den Atemgasstrom einer Atemöffnung des Benutzers zuzuführen, ist eine hier stark schematisiert dargestellte Atemschnittstelle 102 vorgesehen. Die Atemschnittstelle 102 ist vorzugsweise als ein Patienteninterface ausgebildet und kann beispielsweise als eine Vollgesichtsmaske, als ein Nasal-Pillow, als ein Tubus oder als eine Larynxmaske ausgestaltet sein. Die hier gezeigte Atemschnittstelle 102 ist eine als Nasalmaske ausgebildete Atemmaske 105. Zur Fixierung der Atemmaske 105 kann eine Kopfhaube vorgesehen sein.

Zur Verbindung der Atemschnittstelle 102 mit der Beatmungseinrichtung 2 können verschiedene Typen von Schlaucheinrichtungen 25 eingesetzt werden. Die gewünschte Schlaucheinrichtung 25 wird über eine Kupplungseinrichtung 4 an der Beatmungseinrichtung 2 angebunden, sodass die Strömungsverbindung 5 zwischen Gebläseeinrichtung 3 und Atemschnittstelle 102 bereitgestellt wird. Die Schlaucheinrichtung 25 weist an den jeweiligen Enden Kuppelungselemente auf, um an der Atemschnittstelle 102 sowie an der Kupplungseinrichtung 4 angebunden zu werden.

In der Figur 1 ist oben links eine Schlaucheinrichtung 25 zur Leckagebeatmung mit einem Beatmungsschlauch 45 gezeigt. Im Beatmungsschlauch 45 oder in der Atemschnittstelle 102 und beispielsweise in der Atemmaske 105 ist dazu eine definierte Öffnung vorgesehen. Über diese Öffnung werden die ausgeatmete Luft und insbesondere das exspiratorische Kohlendioxid ausgewaschen. Der Beatmungsschlauch 45 wird über eine erste Kupplung 14 der Kupplungseinrichtung 4 mit der Beatmungseinrichtung 2 strömungsverbunden.

Rechts daneben sowie darunter sind zwei Schlaucheinrichtungen 25 gezeigt, welche zur Ventilbeatmung dienen und ein Ausatemventil 101 umfassen. Unten ist eine Schlaucheinrichtung 25 mit einem Beatmungsschlauch 45 und einem Ausatemschlauch 55 gezeigt, ein sogenanntes Zweischlauchsystem. Ein solches Zweischlauchsystem wird beispielsweise dann eingesetzt, wenn das exspiratorische Volumen besonders zuverlässig bestimmt werden muss. Der Ausatemschlauch 55 wird dazu über eine zweite Kupplung 24 mit der Beatmungseinrichtung 2 strömungsverbunden. Über einen Y-Adapter 112 werden die beiden Schläuche 45, 55 mit der Atemschnittstelle 102 strömungsverbunden.

Die beiden oben gezeigten Schlaucheinrichtungen 25 sind sogenannte Einschlauchsysteme und umfassen jeweils einen Beatmungsschlauch 45, jedoch keinen Ausatemschlauch 55.
Die Schlaucheinrichtungen 5 umfassen hier jeweils einen Messschlauch 110, welcher über einen Eingangsstutzen 111 mit der Beatmungseinrichtung 2 strömungsverbunden ist. So können eine charakteristische Größe (6') und beispielsweise der Druck und/oder der Flow der Atemgasströmung patientennah erfasst werden. Dazu befindet sich in der Beatmungseinrichtung beispielsweise wenigstens ein Drucksensor und/oder wenigstens ein Flowsensor, welcher mit dem Eingangsstutzen 111 strömungsverbunden ist. Der Messschlauch 110 bzw. der Eingangsstutzen 111 können hier sowohl zur Überwachung der Beatmung als auch zur Überwachung einer Rückströmung des Ausatemflusses herangezogen werden.

Die Schlaucheinrichtung 25 zur Ventilbeatmung umfasst hier ein steuerbares Patientenventil 101. An das Patientenventil 101 wird die Atemschnittstelle 102 und beispielsweise die Atemmaske 105 angeschlossen. Die Ansteuerung des Patientenventils 101 erfolgt über einen Steuerschlauch 109, welcher strömungsverbunden an dem Patientenventil 101 sowie an einem Steuerstutzen 119 der Beatmungseinrichtung 2 angeschlossen ist. Mit dem Ausatemventil 101 wird die Ein- und Ausatmung gesteuert. Bei einem Einsatz einer Schlaucheinrichtung 25 für eine Ventilbeatmung dürfen in der Regel keine Beatmungszugänge mit Leckageöffnungen verwendet werden.

Der Steuerschlauch 109 wird durch die Beatmungseinrichtung 2 mit einem gezielten Steuerdruck beaufschlagt und beispielsweise mit einem EPAP- oder PEEP-Steuerdruck beaufschlagt. Dadurch kann das Patientenventil 101 zur gewünschten Zeit geöffnet bzw. geschlossen werden.

Die hier gezeigten Schlaucheinrichtungen 25 können je nach Patient bzw. Beatmungsbedarf gegeneinander ausgetauscht werden. Wird eine Schlaucheinrichtung 25 zur Ventilbeatmung eingesetzt, kann es ohne weitere Maßnahmen zu einer unerwünschten Rückatmung in die Beatmungseinrichtung 2 kommen. Zum Schutz der Beatmungseinrichtung 2 vor der rückströmenden Ausatemluft ist hier eine Überwachungseinrichtung 6 vorgesehen. Die Überwachungseinrichtung 6 ist hier Teil der Steuereinrichtung 16 bzw. in diese integriert.

Die Überwachungseinrichtung 6 überwacht den in die Strömungsverbindung 5 zurückströmenden aus Atemfluss des Patienten. Wird ein entsprechender Rückfluss erkannt, der zum Eintreten von Ausatemluft in die Beatmungseinrichtung 2 führen würde, wird ein Staudruck erzeugt. Durch den Staudruck wird einem Eintreten der Ausatemluft in die Beatmungseinrichtung 2 entgegengewirkt. Die Verhinderung des Rückflusses durch den Staudruck wird mit Bezug zu den nachfolgenden Figuren näher beschrieben.

So ist ein Wechsel zwischen einer Leckagebeatmung und einer Ventilbeatmung durch ein einfaches und komfortables Tauschen der Schlaucheinrichtungen 25 möglich, ohne dass Umbauten zur Verhinderung des Rückflusses nötig werden. Besonders vorteilhaft ist, dass das Beatmungsgerät 1 hier kein Rückschlagventil umfasst, welches bei einem Wechsel des Ausatemsystems eingebaut bzw. ausgebaut werden müsste. Ein solches Rückschlagventil darf in der Regel bei einem Leckagesystem nicht eingesetzt werden, damit der Rückfluss während der Exspiration möglich ist.

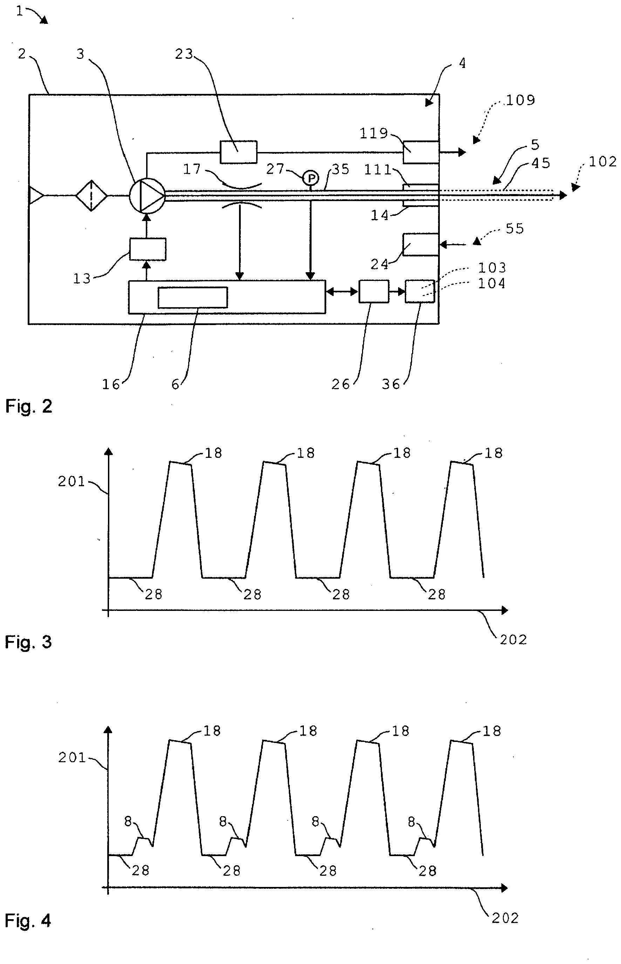

Die Figur 2 zeigt eine sehr schematische Darstellung des Beatmungsgerätes 1. Die Wirkverbindungen zwischen den einzelnen Komponenten sind hier stark schematisiert und vereinfacht als Pfeile dargestellt.

Die Beatmungseinrichtung 2 umfasst eine Gebläseeinrichtung 3 zur Erzeugung der Atemgasströmung. Die Atemgasströmung wird über einen in der Beatmungseinrichtung 2 liegenden Geräteabschnitt 35 zur ersten Kupplung 14 geführt. Von dort wird die Atemgasströmung über einen Beatmungsschlauch 45 weiter zur Atemschnittstelle 102 geleitet. Der Geräteabschnitt 35 und der Beatmungsschlauch 45 stellen hier die Strömungsverbindung 5 zur Verfügung.

Um eine charakteristische Größe (6') für einen Strömungsfluss innerhalb der Strömungsverbindung 5 zu erfassen, sind hier ein Flowsensor 17 und ein Drucksensor 27 vorgesehen. Die charakteristische Größe (6') für den Strömungsfluss ist hier der Volumendurchfluss und/oder der Druck. Der Flowsensor 17 und der Drucksensor 27 sind hier mit der Strömungsverbindung 5 strömungsverbunden, sodass der dort anliegende Druck bzw.

Volumendurchfluss erfassbar ist. Die erfassten Werte für den Druck und den Volumendurchfluss werden der Steuereinrichtung 16 bzw. der Überwachungseinrichtung 6 bereitgestellt, sodass diese die Gebläseeinrichtung 3 entsprechend ansteuern bzw. regeln kann.

Der Flowsensor 17 und der Drucksensor 27 können auch mit dem Eingangsstutzen 111 strömungsverbunden sein, sodass der Druck bzw. Flow auch über einen angeschlossenen Messschlauch 110 erfassbar ist.

Zur Ansteuerung der Gebläseeinrichtung 3 durch die Steuereinrichtung 16 bzw. Überwachungseinrichtung 6 ist hier eine Treibereinrichtung 13 vorgesehen. Die Steuereinrichtung 16 berücksichtigt dabei wenigstens eine Gerätekonfiguration sowie Vorgaben zur Einstellung bestimmter Beatmungsparameter. \
Zur Vornahme von Einstellungen für die Gerätekonfiguration und/oder zur Einstellung von Beatmungsparametern ist hier ein User Interface 36 mit einer Bedieneinrichtung 103 und einer Anzeigeeinrichtung 104 vorgesehen. Die vorgenommenen Eingaben werden über einen User Interface Controller 26 der Steuereinrichtung 16 zur Verfügung gestellt.

Zudem kann die von der Gebläseeinrichtung 3 erzeugte Atemgasströmung auch als Steuerdruck für ein Ausatemventil 101 eingesetzt werden. Dazu wird die Atemgasströmung über den Steuerstutzen 119 und einen daran angeschlossenen Steuerschlauch 109 zum Patientenventil geführt. Um den Steuerdruck gezielt einstellen zu können, ist hier ein Aktor 23 vorgesehen. Dieser stellt beispielsweise den gewünschten EPA- oder PEEP-Steuerdruck ein. Der Aktor 23 ist insbesondere mit der Steuereinrichtung 16 wirkverbunden.

Die Beatmungseinrichtung 2 umfasst hier auch eine zweite Kupplung 24 für einen Ausatemschlauch 55. Die dort einströmende Ausatemluft wird durch die Beatmungseinrichtung 2 in die Umgebung freigesetzt.

Zum Schutz der Beatmungseinrichtung 2 vor rückströmender Ausatemluft überwacht die Überwachungseinrichtung 6 den Strömungsfluss innerhalb der Strömungsverbindung 5. Dazu wird anhand des Flowsensors 17 der Volumendurchfluss erfasst. Zeigt der Volumendurchfluss einen Wert unterhalb eines Schwellenwertes oder wird ein negativer Volumendurchfluss erfasst, regelt die Überwachungseinrichtung 6 die Gebläseeinrichtung 3 auf einen entsprechenden Staudruck.

Dabei kann vorgesehen sein, dass der Staudruck erst nach einer Latenzzeit aufgebaut wird, welche dynamisch festgelegt wird. Die Latenzzeit beschreibt dabei die Zeit, welche der rückströmende Ausatemfluss zum Durchströmen des Beatmungsschlauches 45 benötigt. Dabei berücksichtigt die Überwachungseinrichtung zur Festlegung der Latenzzeit beispielsweise ein hinterlegtes Totraumvolumen des Beatmungsschlauches 45. Zudem berücksichtigt die Überwachungseinrichtung 6 auch den Druck und/oder dem Volumendurchfluss und/oder eine Fließgeschwindigkeit zur Festlegung der Latenzzeit.

So kann der Staudruck gezielt dann aufgebaut werden, wenn der Ausatemfluss den Beatmungsschlauch 45 durchströmt hat, aber noch nicht in das Beatmungsgerät 2 bzw. den Geräteabschnitt 35 eingetreten ist. Die Latenzzeit kann aber auch so gewählt werden, dass der rückströmende Ausatemfluss den Beatmungsschlauch 45 nur teilweise oder gar nicht durchströmt. Die Höhe des Staudrucks wird von der Überwachungseinrichtung hier so eingestellt, dass dieser zum Zurückhalten des Ausatemflusses ausreichend hoch ist, aber die geforderte Beatmung nicht unvorteilhaft beeinflusst. Dabei berücksichtigt die Überwachungseinrichtung 6 wenigstens einen Beatmungsparameter und beispielsweise einen PEEP und/oder einen EPAP. Zur Überwachung bzw. Regelung des Staudrucks kann der durch den Drucksensor 27 erfasste Wert herangezogen werden. Die Überwachungseinrichtung 6 kann die ermittelten Werte für den Volumendurchfluss auch zur Auswertung eines zeitlichen Ablaufs von Inspirations- und Exspirations-Phasen auswerten. So lassen sich anhand der erfassten Größe (6') für den Strömungsfluss auch Aussagen über die Atemfrequenz und/oder den Atemluftbedarf des Patienten machen.

Beim Ausatmen verursacht die von dem Patienten unter einem entsprechenden Druck entgegen der aus der Beatmungseinrichtung 2 zugeführten Atemgasströmung ausgeblasene Atemluft, dass sich in der Strömungsverbindung 5 gegenläufige Gasströme ergeben. Während der Ausatmung verlangsamt sich der Zustrom des frischen Atemgases aus der ' Beatmungseinrichtung 2 bzw. dem Beatmungsschlauch 45. Diese Verlangsamung wird insbesondere durch den Flowsensor 17 sensorisch in einem entsprechend verringerten Volumendurchfluss ermittelt. Die Überwachungseinrichtung 6 erkennt die Rückatmung hier somit anhand eines verringerten Volumendurchflusses.

Die Überwachungseinrichtung 6 ermittelt dann die notwendigen Einstellungen der Gebläseeinrichtung 3 und beispielsweise die notwendige Einstellung der Gebläsedrehzahl. Zur Regelung des Staudrucks kann die Überwachungseinrichtung 6 neben dem erfassten Volumendurchfluss auch den durch den Drucksensor 27 erfassten Druck berücksichtigen. Möglich ist auch, dass die Überwachungseinrichtung 6 die Atemfrequenz und/oder den Atemluftbedarf des Patienten für die Regelung der Gebläseeinrichtung 3 bei der Einstellung des Staudrucks berücksichtigt. So lässt sich ein Staudruck am Ausgang des Geräteabschnitts 35 erzeugen, welcher eine Rückatmung wirkungsvoll verhindert und zugleich eine optimale Beatmungsqualität ermöglicht.

Um einen optimalen Spülfluss gewährleisten zu können, durch welchen die Ausatemluft während der Ausatemphase möglichst vollständig ausgewaschen werden kann, kann hier auch das Ausatemventil 101 durch die Überwachungseinrichtung 6 angesteuert werden. Dabei wird insbesondere ein Öffnungsgrad des Ausatemventils 101 eingestellt. So kann besonders viel der CO2-reichen Ausatemluft ausgewaschen werden. Dazu wird über den Aktor 23 der PEEP-Steuerdruck auf den erforderlichen Öffnungsgrad geregelt.

Die Gebläseeinrichtung 3 wird während der Ausatemphase vorzugsweise so eingestellt, dass eine gewünschte Spülung erreicht wird. Dabei werden insbesondere der Spüldruck und der Öffnungsgrad des Ausatemventils 101 aufeinander abgestimmt. Unter Überwachung des Volumens werden der Öffnungsgrad des Ausatemventils 101 und zeitweise der zweite Beatmungsdruck über die Gebläseeinrichtung 3 so gesteuert, dass mit Beginn der nachfolgenden Einatemphase die die CO2-angereicherte Ausatemluft möglichst vollständig ausgewaschen ist.

In der Figur 3 ist ein stark schematisiert dargestellter Druckverlauf während einer beispielhaften Beatmung gezeigt. Dazu wurde der Druck 201 gegen die Zeit 202 aufgetragen. Während einer Einatemphase des Patienten wird dabei ein erster Beatmungsdruck 18 aufgebaut, um dem Patienten ein erforderliches Volumen an frischem Atemgas unter einem erforderlichen Druck zuzuführen. Während der Ausatemphase wird die Atemgasströmung dann auf einen zweiten Beatmungsdruck eingestellt, welcher den Ausatmungsvorgang des Patienten unterstützt. Die hier gezeigte Beatmung umfasst somit eine zyklische . Änderung des Beatmungsdrucks während der Einatemphase bzw. Ausatemphase. Der zweite Beatmungsdruck wird durch die Überwachungseinrichtung 6 auf einen für die Beatmung geforderten Solldruck eingestellt. Der Solldruck ist beispielsweise auf einen gewünschten PEEP oder einen EPAP eingestellt.

Die Figur 4 zeigt einen stark schematischen Druckverlauf einer anderen Beatmung. Hier wurde nach dem hier vorgestellten beispielhaften Verfahren zum Schutz der Beatmungseinrichtung vor rückströmender Ausatemluft zeitweise ein Staudruck 8 beaufschlagt. Der Staudruck 8 ist hier eine gezielte Erhöhung des zweiten Beatmungsdrucks 28. Die Höhe des Drucks bzw. die Dauer und der Zeitpunkt des Staudrucks 8 werden dabei durch die Überwachungseinrichtung 6 festgelegt. Die Überwachungseinrichtung 6 berücksichtigt dabei insbesondere den erfassten Volumendurchfluss und die geforderten Beatmungsparameter.

In dem hier gezeigten Beispiel wird der zweite Beatmungsdruck 28 gegen Ende der Ausatemphase auf ein höheres Druckniveau angehoben, um den Staudruck 8 bereitzustellen. Dadurch kann der Staudruck 8 gezielt dann aufgebaut werden, wenn der rückströmende Ausatemfluss ein Maß erreicht, das zu einer unerwünschten Einströmung in die Beatmungseinrichtung 2 führen könnte. Alternativ kann der Staudruck bedarfsabhängig und unabhängig von dem ersten oder zweiten Beatmungsdruck eingestellt werden. Der Staudruck (8) kann unabhängig von den Atemphasen dann hochgeregelt werden, wenn die Überwachungseinrichtung 6 eine beginnende oder drohende Rückatmung in das Gerät ermittelt.

Der Staudruck 8 wird hier durch den ersten Beatmungsdruck abgelöst, welcher mit der nächsten Einatemphase beaufschlagt wird. Der Staudruck 8 kann aber auch wieder auf das Druckniveau des zweiten Beatmungsdrucks oder tiefer abgesenkt werden. Dann wird der zweite Beatmungsdruck 28 nach Ende der Ausatemphase durch den ersten Beatmungsdruck abgelöst.

Der Staudruck kann beispielsweise auch höher als der erste und zweite Druck sein. Der erste und zweite Druck können zumindest zeitweise identisch sein.

Die hier vorgestellte Erfindung hat den Vorteil, dass eine Rückatmung während einer Ventilbeatmung nicht durch ein Rückschlagventil verhindert wird, sondern durch die geeignete Regelung der Gebläseeinrichtung 3 bzw. durch den Aktor 23 für den PEEP-Steuerdruck. Dadurch ist ein Wechsel zwischen Leckagebeatmung und Ventilbeatmung besonders bedienerfreundlich und ohne Umbauten möglich.

### Bezugszeichenliste:

- 1: Beatmungsgerät
- 2: Beatmungseinrichtung
- 3: Gebläseeinrichtung
- 4: Kupplungseinrichtung
- 5: Strömungsverbindung
- 6: Überwachungseinrichtung
- 7: Sensoreinrichtung
- 8: Staudruck
- 13: Treibereinrichtung
- 14: Kupplung
- 16: Steuereinrichtung
- 17: Flowsensor
- 18: erster Beatmungsdruck
- 23: Aktor
- 24: Kupplung
- 25: Schlaucheinrichtung
- 26: User Interface Controller
- 27: Drucksensor
- 28: zweiter Beatmungsdruck
- 35: Geräteabschnitt
- 36: User Interface
- 45: Beatmungsschlauch
- 55: Ausatemschlauch
- 100: Heimbeatmungsgerät
- 101: Ausatemventil
- 102: Atemschnittstelle
- 103: Bedieneinrichtung
- 104: Anzeigeeinrichtung
- 105: Atemmaske
- 109: Steuerschlauch
- 110: Messschlauch
- 111: Eingangsstutzen
- 112: Y-Adapter
- 119: Steuerstutzen
- 200: Zeit
- 201: Druck

## Patentansprüche

1. Beatmungseinrichtung (2) zum Schutz eines Beatmungsgerätes (1) vor rückströmender Ausatemluft, wobei die Beatmungseinrichtung (2) wenigstens eine Gebläseeinrichtung (3) umfasst, die eingerichtet ist, eine Atemgasströmung zu erzeugen und über wenigstens eine Strömungsverbindung (5) zu einer Atemschnittstelle (102) zu führen, wobei die Beatmungseinrichtung (2) eingerichtet ist, die Atemgasströmung anhand der wenigstens einer Gebläseeinrichtung (3) während einer Einatemphase des Patienten auf wenigstens einen ersten Beatmungsdruck (18) und während einer Ausatemphase auf wenigstens einen zweiten Beatmungsdruck (28) einzustellen, **dadurch gekennzeichnet, dass** die Beatmungseinrichtung (2) zumindest eine Überwachungseinrichtung (6) umfasst, die eingerichtet ist, zur Überwachung rückströmender Ausatemluft mittels wenigstens einer Sensoreinrichtung (7) wenigstens eine charakteristische Größe (6') für einen Strömungsfluss innerhalb der Strömungsverbindung (5) zu erfassen, wobei die Überwachungseinrichtung (6) eingerichtet ist, wenigsten einen Staudruck (8) gegenüber dem rückströmenden Ausatemfluss durch eine gezielte Erhöhung des Beatmungsdrucks mittels der Gebläseeinrichtung (3) unter Berücksichtigung der erfassten Größe (6') einzustellen, sodass durch den Staudruck (8) einer unerwünschten Rückatmung in die Beatmungseinrichtung (2) entgegengewirkt wird, wobei der Staudruck gleich oder größer als der Druck einer rückströmenden Ausatemluft ist, sodass der Staudruck die Strömung der Ausatemluft in der Strömungsverbindung (5) behindert.

2. Beatmungseinrichtung (2) nach dem vorhergehenden Anspruch, **dadurch gekennzeichnet, dass** die charakteristische Größe (6') für den Strömungsfluss ein Volumendurchfluss ist.

3. Beatmungseinrichtung (2) nach einem der vorhergehenden Ansprüche, **dadurch gekennzeichnet, dass** die Überwachungseinrichtung (6) eingerichtet ist, unter Berücksichtigung der erfassten Größe (6') die Höhe und/oder die Dauer des Staudrucks (8) und/oder des Zeitpunktes der Erzeugung des Staudrucks (8) einzustellen.

4. Beatmungseinrichtung (2) nach einem der vorhergehenden Ansprüche, **dadurch gekennzeichnet, dass** die Überwachungseinrichtung (6) eingerichtet ist, den Staudruck (8) zu beaufschlagen, wenn die erfasste Größe (6') einen Strömungsfluss innerhalb der Strömungsverbindung (5) unterhalb eines Schwellenwertes anzeigt und/oder einen negativen Strömungsfluss anzeigt.

5. Beatmungseinrichtung (2) nach einem der vorhergehenden Ansprüche, **dadurch gekennzeichnet, dass** die Strömungsverbindung eine an die Beatmungseinrichtung (2) koppelbare Schlaucheinrichtung (25) aufweist und die Überwachungseinrichtung (6) eingerichtet ist, den Staudruck (8) nach einer Latenzzeit zu beaufschlagen, wobei die Latenzzeit die Strömungsdauer des rückströmenden Ausatemflusses durch die Schlaucheinrichtung (25) definiert berücksichtigt.

6. Beatmungseinrichtung (2) nach dem vorhergehenden Anspruch 5, **dadurch gekennzeichnet, dass** die Überwachungseinrichtung (6) eingerichtet ist, die Latenzzeit dynamisch zu errechnen und dabei ein in der Überwachungseinrichtung (6) hinterlegtes Totraumvolumen der Schlaucheinrichtung (25) und/oder wenigstens einen Wert des negativen Strömungsflusses zu berücksichtigen.

7. Beatmungseinrichtung (2) nach einem der vorhergehenden Ansprüche, **dadurch gekennzeichnet, dass** die Beatmungseinrichtung (2) in einem Beatmungsgerät (1) angeordnet ist, wobei das Beatmungsgerät (1) in einem Betriebsmodus zur Ventilbeatmung betrieben wird und wobei dazu während der Ausatemphase des Patienten wenigstens ein Ausatemventil (101) zum Abführen von Ausatemluft geöffnet wird.

8. Beatmungseinrichtung nach dem vorhergehenden Anspruch 7, wobei die Überwachungseinrichtung (6) eingerichtet ist, einen Öffnungsgrad des Ausatemventils (101) anzusteuern und wobei der Öffnungsgrad des Ausatemventils (101) und die Einstellung des Staudrucks (8) durch die Überwachungseinrichtung (6) aufeinander abstimmbar sind, sodass eine Spülung der Ausatemluft anpassbar ist.

9. Beatmungseinrichtung (2) nach einem der vorhergehenden Ansprüche 7 oder 8, **dadurch gekennzeichnet, dass** die Überwachungseinrichtung (6) eingerichtet ist, eine Regelung der Einstellung des Staudrucks (8) und/oder eines Öffnungsgrades des Ausatemventils (101) in Echtzeit durchzuführen.

10. Beatmungseinrichtung (2) nach einem der vorhergehenden Ansprüche, **dadurch gekennzeichnet, dass** die Überwachungseinrichtung (6) eingerichtet ist, bei der Einstellung des Staudrucks (8) auch wenigstens einen Beatmungsparameter und insbesondere den Atemluftbedarf und/oder die Atemfrequenz zu berücksichtigen.

11. Beatmungseinrichtung (2) nach einem der vorhergehenden Ansprüche, **dadurch gekennzeichnet, dass** die Überwachungseinrichtung (6) eingerichtet ist, den zweiten Beatmungsdruck (28) auf wenigstens einen für die Ausatemphase geforderten Solldruck für einen PEEP oder einen EPAP einzustellen.

12. Beatmungseinrichtung (2) nach einem der vorhergehenden Ansprüche, **dadurch gekennzeichnet, dass** die Überwachungseinrichtung (6) eingerichtet ist, den zweiten Beatmungsdruck (28) auf den Solldruck zu einem anderen Zeitpunkt als die Anhebung des Beatmungsdrucks auf den Staudruck (8) einzustellen.

13. Beatmungseinrichtung (2) nach einem der vorhergehenden Ansprüche, **dadurch gekennzeichnet, dass** die Überwachungseinrichtung (6) eingerichtet ist, den Solldruck gegenüber dem Staudruck (8) priorisiert einzustellen.

14. Beatmungseinrichtung (2) nach einem der vorhergehenden Ansprüche, **dadurch gekennzeichnet, dass** die Überwachungseinrichtung (6) eingerichtet ist, die Höhe und/oder Dauer und/oder den Zeitpunkt des Staudrucks (8) unter Berücksichtigung des Solldrucks einzustellen.

15. Beatmungseinrichtung (2) nach einem der vorhergehenden Ansprüche, **dadurch gekennzeichnet, dass** die Beatmungseinrichtung (2) in einem Beatmungsgerät (1) angeordnet ist, wobei das Beatmungsgerät (1) in einem Betriebsmodus zur Ventilbeatmung ohne ein Rückschlagventil zur Verhinderung von rückströmender Ausatemluft betrieben wird.

16. Beatmungseinrichtung (2) nach einem der vorhergehenden Ansprüche, **dadurch gekennzeichnet, dass** die Strömungsverbindung (5) durch wenigstens eine an die Beatmungseinrichtung (2) koppelbare Schlaucheinrichtung (25) und durch wenigstens einen in der Beatmungseinrichtung (2) aufgenommen Geräteabschnitt (35) bereitgestellt wird und wobei die Überwachungseinrichtung (6) eingerichtet ist, die charakteristische Größe (6') zu erfassen und/oder den Staudruck (8) im Geräteabschnitt (35) zu beaufschlagen.

17. Beatmungsgerät (1) umfassend wenigstens eine Beatmungseinrichtung (2) nach einem der Ansprüche 1 bis 16.

## Claims

1. A ventilation apparatus (2) for protecting a ventilation device (1) from backflowing expiratory air, wherein the ventilation apparatus (2) comprises at least one fan apparatus (3) that is configured to generate a respiratory gas flow and to guide it via at least one flow connection (5) to a respiratory interface (102), wherein the ventilation apparatus (2) is configured to adjust the respiratory gas flow using the at least one fan apparatus (3) during an inspiratory phase by the patient to at least one first ventilation pressure (18) and to at least one second ventilation pressure (28) during an expiratory phase, **characterized in that** the ventilation apparatus (2) comprises at least one monitoring apparatus (6) that is configured to measure at least one characteristic quantity (6') for a flow within the flow connection (5) to monitor backflowing expiratory air by means of at least one sensor apparatus (7), wherein the monitoring apparatus (6) is configured to adjust at least one stagnation pressure (8) relative to the back-flowing expiratory flow by specifically increasing the ventilation pressure by means of the fan apparatus (3) taking into account the measured quantity (6') so that, by means of the stagnation pressure (8), undesired re-breathing into the ventilation apparatus (2) is counteracted, wherein the stagnation pressure is equal to or greater than the pressure of back-flowing expiratory air so that the stagnation pressure prevents the flow of the expiratory air in the flow connection (5).

2. The ventilation apparatus (2) according to the preceding claim, **characterized in that** the characteristic quantity (6') for the flow is a volumetric flow.

3. The ventilation apparatus (2) according to one of the preceding claims, **characterized in that** the monitoring apparatus (6) is configured to adjust the level and/or the duration of the stagnation pressure (8) and/or the point in time for generating the stagnation pressure (8) taking into account the measured quantity (6').

4. The ventilation apparatus (2) according to one of the preceding claims, **characterized in that** the monitoring apparatus (6) is configured to apply the stagnation pressure (8) when the recorded quantity (6') indicates a flow within the flow connection (5) below a threshold value, and/or indicates a negative flow.

5. The ventilation apparatus (2) according to one of the preceding claims, **characterized in that** the flow connection has a hose apparatus (25) that can be coupled to the ventilation apparatus (2), and the monitoring apparatus (6) is configured to apply the stagnation pressure (8) after a latency period, wherein the latency period takes into account the flow duration of the back-flowing expiratory air through the hose apparatus (25).

6. The ventilation apparatus (2) according to the preceding claim 5, **characterized in that** the monitoring apparatus (6) is configured to dynamically calculate the latency period and, in so doing, take into account a dead space volume of the hose apparatus (25) and/or at least one value of the negative flow saved in the monitoring apparatus (6).

7. The ventilation apparatus (2) according to one of the preceding claims, **characterized in that** the ventilation apparatus (2) is arranged in a ventilation device (1), wherein the ventilation device (1) is operated in an operating mode for valve ventilation, and wherein at least one expiration valve (101) is opened to discharge expiratory air during the expiration phase of the patient.

8. The ventilation apparatus according to the preceding claim 7, wherein the monitoring apparatus (6) is configured to control a degree of opening of the expiratory valve (101), and wherein the degree of opening of the expiratory valve (101) and the adjustment of the stagnation pressure (8) by the monitoring apparatus (6) can be coordinated with each other so that purging the expiratory air is adaptable.

9. The ventilation apparatus (2) according to one of the preceding claims 7 or 8, **characterized in that** the monitoring apparatus (6) is configured to carry out regulation of the adjustment of the stagnation pressure (8) and/or of a degree of opening of the expiratory valve (101) in real time.

10. The ventilation apparatus (2) according to one of the preceding claims, **characterized in that** the monitoring apparatus (6) is configured to also take into account at least one respiration parameter and in particular the required respiratory air and/or the respiratory frequency when adjusting the stagnation pressure (8) .

11. The ventilation apparatus (2) according to one of the preceding claims, **characterized in that** the monitoring apparatus (6) is configured to adjust the second ventilation pressure (28) to at least one target pressure required for the expiratory phase for a PEEP or an EPAP.

12. The ventilation apparatus (2) according to one of the preceding claims, **characterized in that** the monitoring apparatus (6) is configured to adjust the second ventilation pressure (28) to the target pressure at a different time than the elevation of the ventilation pressure to the stagnation pressure (8).

13. The ventilation apparatus (2) according to one of the preceding claims, **characterized in that** the monitoring apparatus (6) is configured to adjust the target pressure prioritized over the stagnation pressure (8).

14. The ventilation apparatus (2) according to one of the preceding claims, **characterized in that** the monitoring apparatus (6) is configured to adjust the level and/or the duration and/or the point in time of the stagnation pressure (8) taking into account the target pressure.

15. The ventilation apparatus (2) according to one of the preceding claims, **characterized in that** the ventilation apparatus (2) is arranged in a ventilation device (1), wherein the ventilation device (1) is operated in an operating mode for valve ventilation without a check valve to prevent back-flowing expiratory air.

16. The ventilation apparatus (2) according to one of the preceding claims, **characterized in that** the flow connection (5) is provided by at least one hose apparatus (25) that can be coupled to the ventilation apparatus (2), and by at least one device section (35) accommodated in the ventilation apparatus (2), and wherein the monitoring apparatus (6) is configured to detect the characteristic quantity (6') and/or apply the stagnation pressure (8) in the device section (35).

17. A respiratory device (1) comprising at least one ventilation apparatus (2) according to one of claims 1 to 16.

## Revendications

1. Dispositif de ventilation (2) destiné à protéger un respirateur (1) du reflux d'air expiré, dans lequel le dispositif de ventilation (2) comprend au moins un dispositif à soufflante (3), qui est ajusté pour générer un flux de gaz respiratoire et l'acheminer à une interface respiratoire (102) par l'intermédiaire d'au moins une connexion fluidique (5), dans lequel le dispositif de ventilation (2) est ajusté pour régler le flux de gaz respiratoire sur au moins une première pression de ventilation (18) à l'aide au moins dudit dispositif à soufflante (3) durant une phase d'inspiration du patient et sur au moins une seconde pression de ventilation (28) durant une phase d'expiration, **caractérisé en ce que** le dispositif de ventilation (2) comprend au moins un dispositif de contrôle (6), qui est ajusté pour surveiller le reflux d'air expiré au moyen d'au moins un dispositif de détection (7), pour détecter au moins une grandeur caractéristique (6') d'un flux s'écoulant à l'intérieur de la connexion fluidique (5), dans lequel le dispositif de contrôle (6) est ajusté pour régler au moins une pression dynamique (8) s'opposant au reflux d'air expiré par une augmentation ciblée de la pression de ventilation au moyen du dispositif à soufflante (3) compté tenu de la grandeur détectée (6'), de sorte qu'une réinspiration indésirable est entravée par la pression dynamique (8) dans le dispositif de ventilation (2), dans lequel la pression dynamique est supérieure ou égale à la pression d'un reflux d'air expiré, de sorte que la pression dynamique empêche que l'air expiré ne s'écoule dans la connexion fluidique (5).

2. Dispositif de ventilation (2) selon la revendication précédente, **caractérisé en ce que** la grandeur caractéristique (6') du flux d'écoulement est un débit volumique.

3. Dispositif de ventilation (2) selon l'une des revendications précédentes, **caractérisé en ce que** le dispositif de contrôle (6) est ajusté pour régler le niveau et/ou la durée de la pression dynamique (8) et/ou l'instant de la création de la pression dynamique (8) compte tenu de la grandeur détectée (6').

4. Dispositif de ventilation (2) selon l'une des revendications précédentes, **caractérisé en ce que** le dispositif de contrôle (6) est ajusté pour appliquer la pression dynamique (8), lorsque la grandeur détectée (6') indique un flux s'écoulant à l'intérieur de la connexion fluidique (5) inférieur à une valeur de seuil et/ou qu'elle indique un flux d'écoulement négatif.

5. Dispositif de ventilation (2) selon l'une des revendications précédentes, **caractérisé en ce que** la connexion fluidique présente un dispositif tubulaire (25) raccordable au dispositif de ventilation (2) et que le dispositif de contrôle (6) est ajusté pour appliquer la pression dynamique (8) après un temps de latence, dans lequel le temps de latence tient compte de la durée d'écoulement du reflux d'air expiré, défini par le dispositif tubulaire (25).

6. Dispositif de ventilation (2) selon la revendication précédente **5, caractérisé en ce que** le dispositif de contrôle (6) est ajusté pour calculer dynamiquement le temps de latence et tenir compte en l'occurrence d'un volume d'espace mort du dispositif tubulaire (25), enregistré dans le dispositif de contrôle (6) et/ou d'au moins une valeur du flux d'écoulement négatif.

7. Dispositif de ventilation (2) selon l'une des revendications précédentes, **caractérisé en ce que** le dispositif de ventilation (2) est disposé dans un respirateur (1), dans lequel le respirateur (1) fonctionne dans un mode opératoire dédié à la ventilation par valve et dans lequel au moins une valve d'expiration (101) est ouverte à cette fin durant la phase d'expiration du patient pour évacuer l'air expiré.

8. Dispositif de ventilation selon la revendication précédente 7, dans lequel le dispositif de contrôle (6) est ajusté pour commander un degré d'ouverture de la valve d'expiration (101) et dans lequel le degré d'ouverture de la valve d'expiration (101) et le réglage de la pression dynamique (8) peuvent être accordés l'un à l'autre par le dispositif de contrôle (6), de sorte qu'un rinçage de l'air expiré est adaptable.

9. Dispositif de ventilation (2) selon l'une des revendications précédentes 7 ou 8, **caractérisé en ce que** le dispositif de contrôle (6) est ajusté pour exécuter une régulation en temps réel du réglage de la pression dynamique (8) et/ou d'un degré d'ouverture de la valve d'expiration (101).

10. Dispositif de ventilation (2) selon l'une des revendications précédentes, **caractérisé en ce que** le dispositif de contrôle (6) est ajusté pour prendre en compte aussi au moins un paramètre de ventilation et notamment le besoin d'air respiratoire et/ou la fréquence respiratoire lors du réglage de la pression dynamique (8) .

11. Dispositif de ventilation (2) selon l'une des revendications précédentes, **caractérisé en ce que** le dispositif de contrôle (6) est ajusté pour régler la seconde pression de ventilation (28) sur au moins une pression de consigne requise pour la phase d'expiration applicable à une PEEP (*pression positive en fin d'expiration*) ou à une EPAP (*pression expiratoire positive dans les voies aériennes*).

12. Dispositif de ventilation (2) selon l'une des revendications précédentes, **caractérisé en ce que** le dispositif de contrôle (6) est ajusté pour régler la seconde pression de ventilation (28) sur la pression de consigne à un autre instant que l'élévation de la pression de ventilation sur la pression dynamique (8).

13. Dispositif de ventilation (2) selon l'une des revendications précédentes, **caractérisé en ce que** le dispositif de contrôle (6) est ajusté pour régler la pression de consigne en priorité par rapport à la pression dynamique (8).

14. Dispositif de ventilation (2) selon l'une des revendications précédentes, **caractérisé en ce que** le dispositif de contrôle (6) est ajusté pour régler le niveau et/ou la durée (8) et/ou l'instant de la pression dynamique (8) compte tenu de la pression de consigne.

15. Dispositif de ventilation (2) selon l'une des revendications précédentes, **caractérisé en ce que** le dispositif de ventilation (2) est disposé dans un respirateur (1), dans lequel le respirateur (1) fonctionne dans un mode opératoire dédié à la ventilation par valve sans aucun clapet anti-retour pour empêcher un reflux d'air expiré.

16. Dispositif de ventilation (2) selon l'une des revendications précédentes, **caractérisé en ce que** la connexion fluidique (5) est obtenue par au moins un dispositif tubulaire (25) raccordable au dispositif de ventilation (2) et par au moins une partie de l'appareil (35) logée dans le dispositif de ventilation (2) et dans lequel le dispositif de contrôle (6) est ajusté pour détecter la grandeur caractéristique (6') et/ou pour appliquer la pression dynamique (8) dans la partie de l'appareil (35).

17. Respirateur (1) comprenant au moins un dispositif de ventilation (2) selon l'une des revendications 1 à 16.
